**Europäisches Patentamt**

**European Patent Office**

**Office européen des brevets**

(11) Veröffentlichungsnummer: **0 110 403**
**A2**

(12) **EUROPÄISCHE PATENTANMELDUNG**

(21) Anmeldenummer: **83112028.2**

(51) Int. Cl.³: **A 61 F 7/00**

(22) Anmeldetag: **30.11.83**

(30) Priorität: **30.11.82 DE 8233638 U**

(43) Veröffentlichungstag der Anmeldung: **13.06.84**
**Patentblatt 84/24**

(84) Benannte Vertragsstaaten: **AT BE CH DE FR GB IT LI LU NL SE**

(71) Anmelder: **Hospipharm Vertriebs GmbH, Gerresheimer-Land-Strasse 110, D-4000 Düsseldorf 12 (DE)**

(72) Erfinder: **Hecht, Manfred, Gerresheimer-Land-Strasse 110, D-4000 Düsseldorf 12 (DE)**

(74) Vertreter: **Ruschke, Hans Edvard et al, Patentanwälte Dipl. Ing. Olaf Ruschke Dipl. Ing. Hans E. Ruschke Dipl.-Ing. Jürgen Rost Dipl.-Chem Dr. U. Rotter Pienzenauerstrasse 2, D-8000 München 80 (DE)**

(54) **Kalt/Heiss-Kompressenanordnung.**

(57) Die Erfindung betrifft eine Kalt/Heiß-Kompressenanordnung, bei der in einem beutelförmigen Gebilde (2, 14') eine Kälte oder Wärme speichernde gelartige Masse enthalten ist. An einer Seite des beutelförmigen Gebildes (2, 14') ist wenigstens eine erste Klettverschlußfläche (21, 22) eines ersten Klettverschlusses befestigt, mit der das beutelförmige Gebilde (2, 14') an der einen Seite einer Bandage (1, 1') befestigbar ist. Die Bandage weist wenigstens eine erste Klettverschlußfläche (11, 10', 11', 12', 13') eines zweiten Klettverschlusses zur Befestigung der Bandage auf, wobei diese erste Klettverschlußfläche des zweiten Klettverschlusses entweder direkt an der Bandage (1, 1') oder an einer zweiten Klettverschlußfläche (12, 13, 14, 15) des zweiten Klettverschlusses befestigbar ist.

- 1 -

----------------------------------------------------------------

Kalt/Heiß-Kompressenanordnung

----------------------------------------------------------------

Die Erfindung betrifft eine Kalt/Heiß-Kompressenanordnung
nach dem Oberbegriff des Patentanspruches 1.

Es sind Kalt/Heiß-Kompressen bekannt, die die Form eines geschlossenen, aus einem Kunststoffmaterial bestehenden Beutels
aufweisen, in dem eine geeignete gelartige Masse enthalten
ist, die Wärme oder Kälte speichern kann. Entsprechend der
gewünschten Behandlung wird der Beutel beispielsweise in
heißem Wasser erhitzt oder im Gefrierfach eines Kühlschrankes abgekühlt und auf die zu behandelnde Körperstelle aufgelegt. Dabei wird vorzugsweise um die aufgelegte Kompresse
ein Handtuch oder dergleichen gewickelt, wobei ein Nachteil
darin besteht, daß eine auf diese Weise an einer Körperstelle befestigte Kompresse leicht verrutschen kann.

Die Aufgabe der vorliegenden Erfindung besteht daher darin, eine Kalt/Heiß-Kompressenanordnung anzugeben, die in einer sehr einfachen und sicheren Weise an Körperstellen unterschiedlicher Umfänge befestigt werden kann.

Diese Aufgabe wird durch eine wie eingangs bereits erwähnte Kalt/Heiß-Kompressenanordnung gelöst, die durch die in dem kennzeichnenden Teil des Patentanspruches 1 angegebenen Merkmale gekennzeichnet ist.

Ein wesentlicher Vorteil der erfindungsgemäßen Kompressenanordnung besteht darin, daß sie aufgrund der Flexibilität des Bandagenbandes dauerhaft an dem gewünschten Körperteil befestigbar ist, ohne daß die Gefahr besteht, daß die Kompresse verrutscht.

Infolge der Anpaßbarkeit des flexiblen Bandagenbandes ist die erfindungsgemäße Kompressenanordnung sehr vielseitig verwendbar.

Vorteilhafterweise ist die erfindungsgemäße Kompressenanordnung wiederverwendbar.

0110403

- 3 -

Ein weiterer wesentlicher Vorteil besteht darin, daß die erfindungsgemäße Kompressenanordnung in einer äußerst einfachen und kostensparenden Weise herstellbar ist.

Im folgenden wird die Erfindung im Zusammenhang mit den Figuren näher erläutert. Es zeigt:

Fig. 1    eine erfindungsgemäße
          Kompressenanordnung;

Fig. 2    eine weitere erfindungsgemäße
          Kompressenanordnung; und

Fig. 3 bis 6 Anwendungen der Kompressenanordnung der Fig. 2.

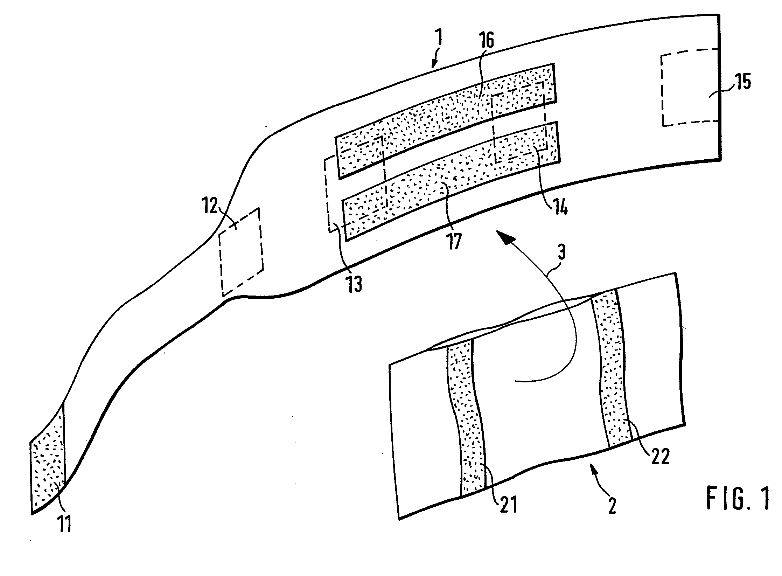

Wie dies aus der Figur 1 ersichtlich ist, besteht die erfindungsgemäße Kompressenanordnung im wesentlichen aus einem Bandagenband 1 und einer Kompresse 2, die vorzugsweise die Form eines aus einem Kunststoffmaterial bestehenden geschlossenen Beutels aufweist, in dem eine Wärme oder Kälte speichernde gelartige Masse enthalten ist. Das Bandagenband 1 besteht vorzugsweise aus einem elastischen Gewebe. In der Figur 1 handelt es sich bei der sichtbaren Seite des elastischen Bandagenbandes 1 um die der zu behandelnden Körperstelle zugewandte Seite. Auf einen vorgegebenen Bereich dieser Seite ist eine Klettverschlußfläche eines ersten Klettverschlusses aufgebracht. Beispielsweise sind in der aus der Figur 1 ersichtlichen Weise als Klettverschlußfläche die streifenförmigen Flächen 16 und 17 des ersten Klettverschlusses aufgebracht. Die entsprechende weitere Klettverschlußfläche dieses ersten Klettverschlusses ist auf einer beutelförmigen Oberfläche der Kompresse 2 angeordnet.

Wie aus der Figur 1 ersichtlich ist, weisen diese auf der Oberfläche der beutelförmigen Kompresse 2 angeordneten weiteren Klettverschlußflächen des ersten Klettverschlusses vorzugsweise die Form von zwei mit dem Bezugszeichen 21 und 22 bezeichneten weiteren Flächen auf. Die Kompresse 2 ist in einer einfachen Weise dadurch an der der zu behandelnden Körperstelle zugewandten Seite des elastischen Bandagenbandes 1 dadurch befestigbar, daß sie in Richtung des Pfeils 3 bewegt wird und so an das Bandagenband 1 angedrückt wird, daß die sich entsprechenden Flächen 16, 17, 21 und 22 des ersten Klettverschlusses wenigstens teilweise zur Deckung gebracht werden und aneinander angreifen. Die Kompresse 2 ist nun verrutschfest und sicher an der der zu behandelnden Körperstelle zugewandten Seite des elastischen Bandagenbandes 1 befestigt.

Das Material des Bandagenbandes 1 kann auch so ausgewählt werden, daß die auf der Kompresse 2 angeordneten Klettverschlußflächen, die hakenförmige Elemente aufweisen, direkt an dem Material des Bandagenbandes 1 haften.

An der Seite des elastischen Bandagenbandes 1, die der der zu behandelnden Körperstelle zugewandten Seite des Bandagenbandes 1 gegenüberliegt, sind entlang der Längsrichtung des Bandagenbandes 1 voneinander beabstandet Klettverschlußflächen 12 bis 15 eines zweiten Klettverschlusses angeordnet. An dem Ende des Bandagenbandes 1 ist wenigstens eine weitere Klettverschlußfläche 11 des zweiten Klettverschlusses, die an einer der Klettverschlußflächen 12 bis 15 des zweiten Klettverschlusses befestigbar ist, auf der Seite des Bandagenbandes 1 angeordnet, die der zu behandelnden Körperstelle zugewandt ist.

Um das elastische Bandagenband 1 mit der darin in der zuvor beschriebenen Weise befestigten Kompresse 2 an einem gewünschten Körperteil zu befestigen, wird zunächst die freie Seite der Kompresse 2 auf die zu behandelnde Körperstelle aufgelegt. Anschließend wird das elastische Bandagenband 1 um das entsprechende Körperteil, bei dem es sich beispielsweise um einen Oberschenkel, einen Arm, oder den Bauch handeln kann, herumgeführt und dadurch befestigt, daß die
Klettverschlußfläche 11 des zweiten Klettverschlusses
an einer der Klettverschlußflächen 12 bis 15 des zweiten
Klettverschlusses befestigt wird. Dabei wird durch die Auswahl einer der Klettverschlußflächen 12 bis 15 des zweiten
Klettverschlusses in Abhängigkeit von dem betreffenden Körperteil die Spannung bestimmt, mit der das elastische Bandagenband 1 die Kompresse 2 gegen die zu behandelnde Körperstelle preßt.

Wie bereits erwähnt, kann das Bandagenband 1 auch aus einem
Material gefertigt sein, das es ermöglicht, daß die Klettverschlußfläche 11 oder die Klettverschlußfläche 15 des
zweiten Klettverschlusses, die hakenförmige Elemente besitzt, direkt an dem Bandagenband befestigbar bzw. verriegelbar ist.

Um während der Behandlung die gegenbenenfalls abgekühlte
bzw. erwärmte Kompresse 2 wieder zu erwärmen bzw. abzukühlen, wird das geschlossene elastische Bandagenband 1 dadurch geöffnet, daß die Klettverschlußfläche 11 des zweiten
Klettverschlusses von einer der Klettverschlußflächen 12
bis 15 des zweiten Klettverschlusses gelöst wird und daß
die Kompresse 2 dadurch von dem elastischen Bandagenband 1 entfernt wird, daß die aneinander angreifenden Klettverschlußflächen 16, 17, 21 und 22 des ersten Klettverschlusses
voneinander getrennt werden.

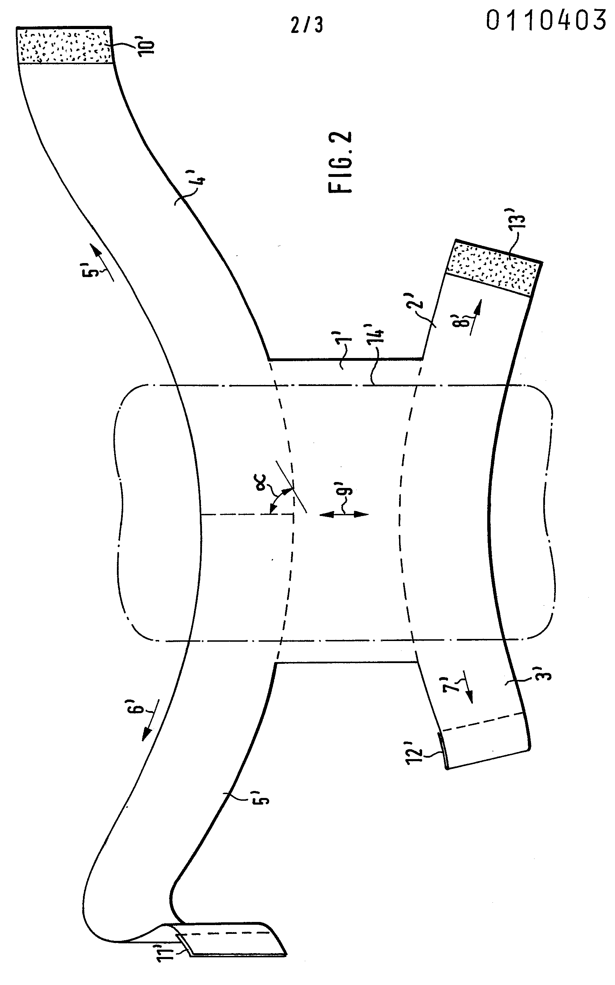

Figur 2 zeigt eine weitere Ausführungsform der erfindungsgemäßen Kompressenanordnung.

Der Vorteil dieser Kompressenanordnung besteht darin,
daß mit ihrer Hilfe das die gelartige Masse enthaltende
beutelförmige Gebilde schnell, mühelos und rutschfest an
verschiedenen Körperstellen, insbesondere im Bereich der
großen Gelenke derart befestigbar ist, daß ein Patient zum
Zwecke der Therapie Bewegungen ausführen kann.

Ein wesentlicher Vorteil besteht darin, daß es durch diese
Kompressenanordnung erstmals ermöglicht wird, während der
Kälte- oder Wärmeeinwirkung gleichzeitig eine krankengymnastische Behandlung im Bereich der großen Gelenke durchzuführen.

Vorteilhafterweise ist ein und dieselbe Größe dieser Kompressenanordnung an alle Körpergrößen anpaßbar. Derartige
Kompressenanordnungen können daher vorteilhafterweise sowohl für Kinder als auch für Erwachsene verwendet werden.

Vorteilhafterweise läßt sich diese Kompressenanordnung mühelos und schnell anlegen und körpergerecht und verrutschsicher befestigen.

Da das Schmerzempfinden eines Patienten durch Kälteeinwirkung reduziert wird, und da diese Kompressenanordnung so an bestimmten Körperbereichen, insbesondere im Bereich der großen Gelenke, befestigbar ist, daß der Patient zum Zwecke der Therapie die durch die Kompressenanordnung erfaßten Bereiche des Körpers bewegen kann, können Muskeln und Gelenke des Patienten während einer krankengymnastischen Therapie vorteilhafterweise nicht nur länger behandelt, sondern auch stärker belastet werden. Unter Kälteeinwirkung ist z.B. die Therapie nach der PNF-Methode besonders zu empfehlen.

Die Schmerzreduzierung bei Kälte und die durch diese Kompressenanordnung ermöglichte größere Mobilität des Patienten führen vorteilhafterweise zu einer erhöhten Kooperationsbereitschaft, wodurch der Rehabilitationsprozeß beschleunigt und der Therapieerfolg günstig beeinflußt werden.

Wie dies aus der Figur 2 ersichtlich ist, weist die erfindungsgemäße Kompressenanordnung ein Grundteil 1' auf,

- 9 -

das eine so große Fläche aufweist, daß es beim Anlegen der
Kompressenanordnung wenigstens einen Teilbereich des die
gelartige Masse enthaltenden beutelförmigen Gebildes überdecken kann. In der Figur 1 ist das beutelförmige Gebilde
14' schematisch durch eine strichpunktierte Linie dargestellt. Zur Befestigung des Gebildes 14' weist dieses
vorzugsweise an seiner dem Grundteil 1' zugewandten Seite
Klettverschlußflächen auf, mit denen das Gebilde 14 am
Grundteil 1' befestigbar ist. Dabei weisen die Klettverschlußflächen am Gebilde  14' hakenförmige Elemente auf,
während das Material des Grundteiles 1' die Ösenfunktion
erfüllen. Bei anderen Materialien des Grundteiles können
an diesem auch eigene, die Ösenfunktion erfüllende Klettverschlußflächen vorgesehen werden.

An sich gegenüberliegenden Seiten des Grundteiles 1' sind
voneinander beabstandet jeweils ein erster Befestigungslappen und ein zweiter Befestigungslappen derart angesetzt, daß sich die Befestigungslappen von dem Grundteil 1'
nach außen erstrecken. Genauer gesagt sind an der einen
Seite, in der Figur 2 beispielsweise an der rechten Seite,
ein erster Befestigungslappen 2' von einem zweiten Befestigungslappen 4' beabstandet angeordnet. In entsprechender
Weise sind an der gegenüberliegenden Seite, d.h. in der

Figur 2 an der linken Seite, des Grundteils 1' ein weiterer erster Befestigungslappen 3' und ein weiterer Befestigungslappen 5' derart angeordnet, daß der weitere erste Befestigungslappen 3' dem ersten Befestigungslappen 2' im wesentlichen gegenüberliegt und daß der weitere zweite Befestigungslappen 5' dem zweiten Befestigungslappen 4' im wesentlichen gegenüberliegt.

Vorzugsweise weisen die ersten Befestigungslappen 2', 3', die hauptsächlich zur Befestigung an einen verhältnismäßig kleinen Umfang aufweisenden Körperteil, wie beispielsweise einem Arm oder einem Bein, gedacht sind, eine kleinere Länge auf als die zweiten Befestigungslappen 4', 5', die zur Befestigung an anderen Körperbereichen, wie beispielsweise am Brustkorb oder an der Taille, bestimmt sind. Die ersten Befestigungslappen 2', 3' weisen an ihren jeweiligen außenliegenden Endbereichen Klettverschlußflächen 12', 13' auf, wobei die eine Klettverschlußfläche an der einen Seite des ihr zugeordneten ersten Befestigungslappens und die andere Klettverschlußfläche an der der einen Seite gegenüberliegenden Seite des ihr zugeordneten ersten Befestigungslappens angebracht ist. In der entsprechenden Weise sind an den äußeren Endbereichen der zweiten Befestigungslappen Klettverschlußflächen 10', 11' an gegenüberliegenden Seiten angebracht. Jede Klettverschlußfläche ist so ausgebildet, daß sie bei der Befestigung des ihr zugeordneten Befestigungslappens an dem jeweils gegenüberliegenden Befestigungslappen an dem Bereich des ihr gegenüberliegenden Befestigungslappens befestigbar ist, auf den sie bei der Befestigung aufgepreßt wird. Dadurch, daß diese Klettverschlußflächen an gegenüberliegenden Seiten der Befestigungslappen angeordnet sind, wird vorteilhafterweise erreicht, daß die Kompressenanordnung an jeder Körperseite eines Patienten rechts oder links angelegt werden kann.

Um einen besonders guten Sitz der Kompressenanordnung an einem vorgegebenen Körperbereich zu erzielen, sind die ersten Befestigungslappen elastisch so ausgebildet, daß sie in ihren Längsrichtungen (Richtung der Pfeile 7', 8') dehnbar sind. In der entsprechenden Weise sind die zweiten Befestigungslappen in ihren Längsrichtungen (Richtung der Pfeile 5', 6') dehnbar. Das Grundteil 1' ist vorzugsweise derart elastisch ausgebildet, daß es in die Richtungen der Pfeile 9' elastisch dehnbar ist.

Vorzugsweise wird die erfindungsgemäße Kompressenanordnung dadurch hergestellt, daß die ersten und zweiten Befestigungslappen an einem in Richtung der Pfeile 9' elastisch dehnbaren Grundteil 1' vernäht werden. Vorzugsweise wird dabei zur Bildung der ersten Befestigungslappen 2', 3' ein bandförmiges Gebilde an dem unteren Bereich des Grundteiles 1' vernäht, dessen Enden die erste Befestigungslappen bilden. In der entsprechenden Weise kann zur Bildung der zweiten Befestigungslappen ein bandförmiges Gebilde an der oberen Seite des Grundteils 1' vernäht werden.

Um eine besonders körpergerechte Anordnung der Bandagenanordnung an verschiedenen Körperteilen zu ermöglichen, sind die zweiten Befestigungslappen 4', 5' so am Grundteil 1' befestigt, daß ihre Längsachsen vom oberen Ende eines Grundteiles 1' jeweils schräg nach außen und oben verlaufen. In diesem Fall besteht jeder zweite Befestigungslappen 4' bzw. 5' jeweils aus einem bandförmigen Gebilde, wobei die einander zugewandten Enden dieser bandförmigen Gebilde an der mittleren Längsachse des Grundteiles 1' aneinander vernäht sind.

Bei einer bevorzugten Ausführungsform weist das Grundteil 1' eine Fläche auf, die etwa 29 x 18 cm groß ist. Die Breite der ersten Befestigungslappen und der zweiten Befestigungslappen beträgt bei diesem Ausführungsbeispiel etwa 8 cm. Die Längsachsen der ersten Befestigungslappen verlaufen

- 13 -

etwa senkrecht zur Längsachse des Grundteiles 1' und die
ersten Befestigungslappen  ragen etwa 12 cm über die Seiten
des Grundteiles 1' hinaus. Die an den Endbereichen der ersten
Befestigungslappen angeordneten Klettverschlußflächen weisen
eine Fläche von etwa 3,5 x 8 cm auf.

Die zweiten Befestigungslappen ragen bei diesem Ausführungsbeispiel etwa 42 cm über das Grundteil 1' hinaus. Vorzugsweise bilden die Längsachsen der zweiten Befestigungslappen
zur Längsachse des Grundteiles 1' einen Winkel $\alpha$ von etwa
70°.

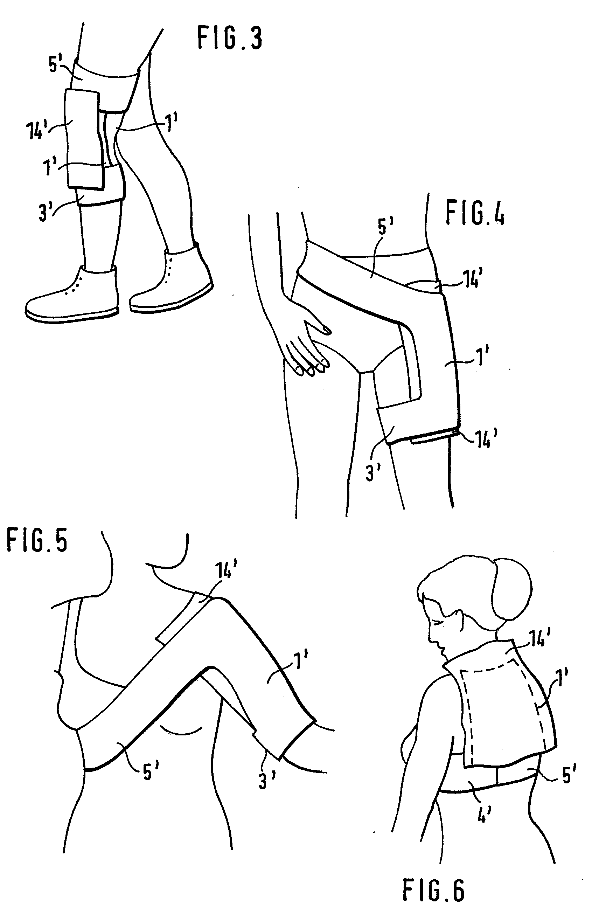

Im Zusammenhang mit den Figuren 3 bis 6 wird im folgenden
erläutert, wie die erfindungsgemäße Kompressenanordnung an
einzelnen Körperstellen im Bereich großer Gelenke befestigt
wird. Die Figur 3 zeigt die Art der Befestigung der Kompressenanordnung am Kniebereich eines Patienten. Dabei
werden vorzugsweise die ersten Befestigungslappen 2', 3',
die kürzer sind, unterhalb des Knies um den einen relativ
kleinen Umfang aufweisenden Wadenbereich herumgewickelt. In
der entsprechenden Weise werden oberhalb des Knies die
zweiten Befestigungslappen 4', 5' um den einen relativ
großen Umfang aufweisenden Oberschenkelbereich herumgewickelt und aneinander befestigt. In der aus der Figur 3
ersichtlichen Weise kann nun das die gelartige Masse enthaltende beutelförmige Gebilde 14', das entweder als Kälte- oder Wärmespeicher dient, mit der Hilfe der an ihm angeordneten Klettverschlußflächen von außen her an dem Grundteil 1' befestigt werden. Das Gebilde 14' bleibt auch dann fest an dem Grundteil 1' haften, wenn der Patient Bewegungen des Kniegelenkes ausführt.

Aus der Figur 4 ist ersichtlich, in welcher Art die erfindungsgemäße Kompressenanordnung im Bereich eines Hüftgelenkes angeordnet werden kann. Zu diesem Zweck werden die ersten Befestigungslappen 2', 3 um den einen relativ kleinen Umfang aufweisenden Oberschenkelbereich derart herumgewickelt und aneinander befestigt, daß das Grundteil 1' der Bandagenanordnung außen am Hüftbereich des Patienten anliegt, wobei zwischen dem Hüftbereich des Patienten und dem Grundteil 1' das beutelförmige Gebilde 14' angeordnet ist. Die zweiten Befestigungslappen 4', 5' werden vom oberen Ende des Grundteiles 1' ausgehend zur gegenüberliegenden Seite des Patienten geführt und dort etwa um den Taillenbereich herumgewickelt und aneinander befestigt. Bei einer derartigen befestigten Bandagenanordnung ist der Patient relativ beweglich.

Aus der Figur 5 ist ersichtlich, wie die erfindungsgemäße Kompressenanordnung im Bereich des Schultergelenks befestigbar ist. Dabei werden die ersten Befestigungslappen 2',
3' um den einen relativ kleinen Umfang aufweisenden Oberarm derart herumgewickelt und aneinander befestigt, daß
das Grundteil 1' an der Außenseite des Oberarmes und auf
dem Schultergelenk aufliegt. Das beutelförmige Gebilde 14'
befindet sich dabei zwischen dem Oberarm bzw. Schultergelenk
und dem Grundteil 1'. Die zweiten Befestigungslappen 4',
5' werden vom oberen Ende des Grundteiles 1' aus zur gegenüberliegenden Körperseite, beispielsweise zum Taillenbereich des Patienten geführt und aneinander befestigt.
Bei einer derartigen Anordnung der Bandagenanordnung können der Oberarm und das Schultergelenk relativ frei bewegt
werden.

Schließlich geht aus der Figur 6 eine Befestigungsart der
Kompressenanordnung am Rücken eines Patienten hervor. Dabei wird die Kompressenanordnung so angelegt, daß das
Grundteil 1' auf dem Rückenbereich des Patienten aufliegt.
Die zweiten Befestigungslappen 4', 5' werden unter den Armen
des Patienten hindurchgeführt und im Brustbereich aneinander befestigt. Die ersten Befestigungslappen (nicht dargestellt) werden über die Schulter des Patienten geführt
und an dem zweiten Befestigungslappen befestigt. Das beu-

- 16 -

telförmige Gebilde 14' wird mit der Hilfe der an ihm vorgesehenen Klettverschlußflächen an der Außenseite des Grundteiles 1' der Kompressenanordnung befestigt.

Vorzugsweise besteht die erfindungsgemäße Bandagenanordnung aus einem Baumwollgewebe-Polyamid-Gemisch, insbesondere aus etwa 22 % Baumwollgewebe und 78 % Polyamid. Um eine Atmung der Bandage zu ermöglichen, sind vorzugsweise einzelne Gewebe-Polyamid-Streifen voneinander beabstandet aneinandergesetzt.

v.Pk./bm

**MÜNCHEN**
Pienzenauerstraße 2
8000 München 80
Telefon: (0 89) 98 03 24,
98 72 58, 98 88 00
Telecopy Gr. II: (0 89) 222 066
Kabel: Quadratur München
Telex: 522 767 quam d

**BERLIN**
Kurfürstendamm 182/183
1000 Berlin 15
Telefon: (0 30) 8 83 70 78/79
Kabel: Quadratur Berlin

Dr.-Ing. Hans Ruschke 1911-1980
Dipl.-Ing. Hans E. Ruschke
Dipl.-Ing. Claf Ruschke*
Dipl.-Ing. Jürgen Post
Dipl.-Chem. Dr. Ulrich Rotter
Patentanwälte
Zugelassen beim Europäischen Patentamt
Admitted to the European Patent Office
* in Berlin

Rainer Schulenberg
Rechtsanwalt
Zugelassen bei den LG München I und II,
beim OLG München und dem
Bayer. Obersten Landesgericht

**RUSCHKE & PARTNER
ANWALTSSOZIETÄT**

München, den 30. Nov. 1983

H 824 HN

HOSPIPHARM Vetriebs-GmbH

Gerresheimer Landstr. 11o, 4ooo Düsseldorf 12

----------------------------------------------------------------

P a t e n t a n s p r ü c h e

1. Kalt/Heiß-Kompressenanordnung, bei der in einem beutelförmigen Gebilde eine Kälte oder Wärme speichernde gelartige Masse enthalten ist, dadurch gekennzeichnet, daß an einer Seite des beutelförmigen Gebildes (2, 14') wenigstens eine erste Klettverschlußfläche (21, 22) eines ersten Klettverschlusses befestigt ist, mit der das beutelförmige Gebilde (2, 14') an der einen Seite einer Bandage (1, 1') befestigbar ist, und daß an der Bandage wenigstens eine erste Klettverschlußfläche (11, 10', 11', 12', 13') eines zweiten Klettverschlusses zur Befestigung der Bandage vorgesehen ist.

2.    Anordnung nach Anspruch 1, dadurch gekennzeichnet, daß an der anderen oder einen Seite der bandförmigen Bandage (1) wenigstens eine zweite Klettverschlußfläche (12 bis 15) eines zweiten Klettverschlusses angeordnet ist, und daß im Bereich eines Endes des Bandagenbandes (1) auf der einen oder anderen Seite des Bandagenbandes (1) die der zweiten Klettverschlußfläche (12 bis 15) des zweiten Klettverschlusses zugeordnete erste Klettverschlußfläche (11) des zweiten Klettverschlusses angeordnet ist (Fig. 1).

3.    Anordnung nach Anspruch 2, dadurch gekennzeichnet, daß die erste Klettverschlußfläche (21, 22) des ersten Klettverschlusses aus zwei zueinander parallel verlaufenden, voneinander beabstandeten streifenförmigen Flächen besteht (Fig. 1).

4.    Anordnung nach Anspruch 2 oder 3, dadurch gekennzeichnet, daß die der ersten Klettverschlußfläche (21, 22) des ersten Klettverschlusses zugeordnete zweite Klettverschlußfläche des ersten Klettverschlusses die Form von zwei sich in Längsrichtung des Bandagenbandes (1) erstreckenden, voneinander beabstandeten streifenförmigen weiteren Flächen (16, 17) aufweist (Fig. 1).

5.    Anordnung nach Anspruch 4, dadurch gekennzeichnet, daß die Längen der den ersten Klettverschlußflächen (21, 22) des ersten Klettverschlusses zugeordneten streifenförmigen weiteren Flächen (16, 17) des ersten Klettverschlusses größer sind als der Abstand zwischen den Außenkanten der streifenförmigen ersten Flächen (21, 22) des ersten Klettverschlusses (Fig. 1).

6.    Anordnung nach einem der Ansprüche 2 bis 5, dadurch gekennzeichnet, daß an der anderen Seite des Bandagenbandes (1) in der Längsrichtung des Bandagenbandes (1) voneinander beabstandet mehrere zweite Klettverschlußflächen (12 bis 15) des zweiten Klettverschlusses angeordnet sind (Fig. 1).

7.    Anordnung nach Anspruch 1, dadurch gekennzeichnet, daß die Bandage ein das beutelförmige Gebilde (14') wenigstens teilweise überdeckendes Grundteil (1') aufweist, das an einer Seite des Grundteiles (1') voneinander beabstandet ein erster Befestigungslappen (2') und ein zweiter Befestigungslappen (4') befestigt sind, daß an der der einen Seite gegenüberliegende Seite des Grundteiles (1') ein weiterer erster Befestigungslappen (3') und ein weiterer zweiter Befestigungslappen (5') voneinander beabstandet angeordnet sind (Fig. 2).

8.    Anordnung nach Anspruch 7, dadurch gekennzeichnet, daß sich der erste Befestigungslappen (2') und der  weitere erste Befestigungslappen (3') sowie der zweite Befestigungslappen (4')
und der weitere zweite Befestigungslappen (5') jeweils gegenüberliegen (Fig. 2).

9.   Anordnung nach Anspruch 7 oder 8, dadurch gekennzeichnet,
daß der erste Befestigungslappen (2') und der weitere erste
Befestigungslappen (3') eine kürzere Länge aufweisen als der
zweite Befestigungslappen (4') und der weitere zweite Befestigungslappen (5') (Fig. 2).

10.  Anordnung nach einem der Ansprüche 7 bis 9, dadurch
gekennzeichnet, daß am Endbereich des ersten Befestigungslappen (2') an der einen Seite der Bandage eine erste
Klettverschlußfläche (13) angeordnet ist, daß am Endbereich
des weiteren ersten Befestigungslappens (3) an der anderen
Seite der Bandage eine zweite Klettverschlußfläche (12')
angeordnet ist, daß am Endbereich des zweiten Befestigungslappens (4') an der einen oder anderen Seite der Bandage
eine dritte Klettverschlußfläche (10') angeordnet ist und
daß am Endbereich des weiteren zweiten Befestigungslappens
(5') an der anderen oder einen Seite der Bandage eine vierte
Klettverschlußfläche (11') angeordnet ist.

11.  Anordnung nach einem der Ansprüche 7 bis 10, dadurch
gekennzeichnet, daß sich die Längsachsen des ersten Befestigungslappens (2') und des zweiten Befestigungslappens (3')
etwa senkrecht zu den Kanten des Grundteiles (1') erstrecken.

12. Anordnung nach einem der Ansprüche 7 bis 11, dadurch
gekennzeichnet, daß die Längsachsen der zweiten Befestigungslappen (4', 5') unter einem spitzen Winkel $\alpha$ derart zu der
zu den Kanten parallelen Mittellinie des Grundteiles (1')
verlaufen, daß sich die zweiten Befestigungslappen (4',5')
von den ersten Befestigungslappen (2', 3') wegerstrecken.

13. Anordnung nach einem der Ansprüche 7 bis 12, dadurch
gekennzeichnet, daß die Fläche des Grundteiles (1') etwa
29 x 18 cm groß ist, wobei die Länge der Kanten des Grundteiles (1') etwa 29 cm betragen, daß die ersten Befestigungslappen (2', 3') etwa 12 cm über die Kanten des Grundteiles
(1') hinausragen und daß die zweiten Befestigungslappen
etwa 42 cm über die Kanten des Grundteiles (1') hinausragen.

14. Anordnung nach Anspruch 11 oder 13, dadurch gekennzeichnet, daß der Winkel $\alpha$ etwa 70° beträgt.

15. Anordnung nach einem der Ansprüche 7 bis 14, dadurch
gekennzeichnet, daß der erste Befestigungslappen (2') und
der weitere erste Befestigungslappen (3') die Form eines
einstückigen Bandes aufweisen, das an dem Grundteil (1')
vernäht ist.

16. Anordnung nach einem der Ansprüche 7 bis 15, <u>dadurch</u> <u>gekennzeichnet, daß</u> der zweite Befestigungslappen (4') und der weitere zweite Befestigungslappen (5') jeweils die Form eines an dem Grundteil (1') vernähten Bandes aufweist.

17. Anordnung nach einem der Ansprüche 1 bis 16, <u>dadurch</u> <u>gekennzeichnet, daß</u> die Bandage (1 bzw. 1' bis 5') aus einem elastischen Material besteht.

18. Anordnung nach Anspruch 17, <u>dadurch gekennzeichnet,</u> daß die Bandage (1 bzw. 1' bis 5') aus einem Baumwollgewebe-Polyamid-Gemisch besteht.

19. Anordnung nach Anspruch 18, <u>dadurch gekennzeichnet,</u> daß die Bandage aus einem Gemisch mit 22 % Baumwollgewebe und 78 % Polyamid besteht.

20. Anordnung nach Anspruch 18 oder 19, <u>dadurch gekenn-</u> <u>zeichnet, daß</u> die ersten und zweiten Befestigungslappen (2' bis 5') jeweils in ihrer Längsrichtung elastisch sind.

21. Anordnung nach Anspruch 20, <u>dadurch gekennzeichnet,</u> daß das Grundteil (1') in Richtung seiner Kanten elastisch ist.

22. Anordnung nach einem der Ansprüche 1 bis 20, dadurch gekennzeichnet, daß die Klettverschlußflächen des zweiten Klettverschlusses jeweils an dem Material der Bandage (1 bzs. 1' bis 5') vernäht sind.

23. Anordnung nach einem der Ansprüche 1 bis 22, dadurch gekennzeichnet, daß die Klettverschlußflächen des ersten Klettverschlusses an dem beutelförmigen Gebilde jeweils verklebt sind.

24. Anordnung nach einem der Ansprüche 1 bis 23, dadurch gekennzeichnet, daß die Klettverschlußflächen des ersten Klettverschlusses und die Klettverschlußflächen des zweiten Klettverschlusses hakenförmige Elemente aufweisen, die an dem Material der Bandage (1 bzw. 1' bis 5') verriegelbar sind.

v.Pk./bm

0110403

FIG. 1

FIG. 2

FIG.3

5'

14'

1'

1'

3'

FIG.4

5'

14'

1'

14'

3'

FIG.5

14'

1'

5'

3'

14'

1'

5'

4'

FIG.6